(19) Europäisches Patentamt
European Patent Office
Office européen des brevets

(11) Veröffentlichungsnummer : 0 312 076 B1

(12) EUROPÄISCHE PATENTSCHRIFT

(45) Veröffentlichungstag der Patentschrift : 25.09.91 Patentblatt 91/39

(21) Anmeldenummer : 88117087.2

(22) Anmeldetag : 14.10.88

(51) Int. Cl.5 : A61K 31/505, C07D 487/14, C07D 495/22, // (C07D487/14, 221:00, 249:00, 239:00), (C07D495/22, 333:00, 249:00, 239:00, 221:00)

# (54) Verwendung von Oxochinazolinderivaten bei der Behandlung der Hyperlipidämie.

(30) Priorität : 15.10.87 DE 3734909

(43) Veröffentlichungstag der Anmeldung : 19.04.89 Patentblatt 89/16

(45) Bekanntmachung des Hinweises auf die Patenterteilung : 25.09.91 Patentblatt 91/39

(84) Benannte Vertragsstaaten : ES

(56) Entgegenhaltungen :
ARZNEIM.-FORSCHUNG./DRUG RES., Band 36(11), Nr. 11, 1986, Seiten 1637-1641; K. TSURUMI et al.: "Effect of the new antiallergic drug 11-oxo-11H-pyrido[2,1-b] quinazoline-2-carboxylic acid on inflammatory reactions and platelet aggregation"

(56) Entgegenhaltungen :
ATHEROSCLEROSIS, Band 5, 1980, Seiten 772-775; R. PAOLETTI et al.: "Prostaglandins, thrombin receptors and platelet aggregation in normal and hypercholesterolemic subjects"
J. PHARM. PHARMACOL., Band 39, Nr. 11, 5. März 1987, Seiten 904-911; K. SEKI et al.: "Influence of the new antihypertensive drug, SM-2470 (a quinazoline derivative), on cholesterol metabolism in rats"

(73) Patentinhaber : BOEHRINGER INGELHEIM KG
Postfach 200
W-6507 Ingelheim am Rhein (DE)

(72) Erfinder : Rauber, Gerhard, Dr.
Magdeburgerstrasse 34
W-6507 Ingelheim (DE)
Erfinder : Stechert, Roland
Dr. Johannes Kohl Strasse 16
W-6530 Bingen am Rhein (DE)
Erfinder : Schromm, Kurt, Dr.
In der Dörrwiese 35
W-6507 Ingelheim am Rhein (DE)

EP 0 312 076 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft die Verwendung bestimmter bekannter Oxochinazolinderivate bei der Behandlung der Hyperlipidämie.

Die Arteriosklerose spielt bei der Genese verschiedener Krankheitsbilder eine ursächliche Rolle. So z.B. bei der Entstehung der koronaren Herzkrankheit, des Herzinfarktes sowie peripherer Zirkulationsstörungen.

Als eine der Hauptursachen wird hierbei die Hyperlipidämie, insbesondere die Hypercholesterinämie angesehen. Zur Behandlung einer derartigen Erhöhung der Blutfettwerte sind zahlreiche sogenannte Lipidsenker im Handel. Diese gliedern sich im wesentlichen in 3 Gruppen :

1. Resorbierbare Antilipidämika (z.B. Clofibrinsäure und ihre Derivate).
2. Nicht resorbierbare Antilipidämika (z.B. Cholestyramin und Sitosterin).
3. Nikotinsäure-Präparate.

Erwartet werden kann bei Anwendung der Lipidsenker im allgemeinen eine Verminderung des Gesamtcholesteringehaltes um ca. 20-25%. Dabei ergibt sich folgendes Nebenwirkungsprofil für die Substanzgruppen:

Zu 1 : Resorbierbare Antilipidämika

Häufiger tritt bei Einnahme dieser Präparate Völlegefühl, Übelkeit, Erbrachen oder Diarrhoe auf. Des weiteren wurde eine Neigung zu ventrikulären Arrhythmien beschrieben. In seltenen Fällen kann ein sogenanntes Muskelsyndrom möglich sein, das mit einem Anstieg der Kreatinin-Phosphokinase- und Transaminase-Werte im Serum sowie entsprechenden EMG-Veränderungen einhergeht. Ferner wurde bei Clofibrat über Fälle von akutem Nierenversagen bei niereninsuffizienten Patienten berichtet. Ursache soll eine mangelnde Dosisanpassung der überwiegend renal ausgeschiedenen Substanz gewesen sein. Des weiteren wurden in dieser Substanzklasse Anstiege der Serumkreatininwerte beobachtet, die eine mögliche nephrotoxische Wirkung in Betracht kommen lassen. Bei Landzeitbehandlung fiel ein Abfall der alkalischen Phosphatase auf ; die Bedeutung dieses Befundes ist noch offen. Es wurde über Granulozytopenie berichtet sowie gelegentliche Potenzstörungen. Auch allergische Exantheme scheinen häufiger als ursprünglich angenommen aufzutreten.

Zu 2 : Nicht resorbierbare Antilipidämika

Eine der unangenehmsten Eigenschaften dieser Substanzgruppe ist das häufige Auftreten von Obstipationen in bis zu 18% der Fälle und mehr. Dies führt bei den meisten Patienten zu einem Abbruch der Therapie. Weiterhin können Völlegefühl, Flatulenz und Übelkeit auftreten. Bei höherer Dosierung kommt es sehr häufig zu Steatorrhoen. Da es sich bei diesen Medikamenten um Ionenaustauscherharze handelt, kommt es gehäuft zu Störungen der Resorption von Medikamenten oder anderen notwendigen Substanzen, wie z.B. Vitamin A, B12, K sowie Folsäure. Des weiteren wurde auch eine hyperchlorämische Azidose bei Patienten mit eingeschränkter Nierenfunktion beschrieben.

Zu 3 : Nikotinsäure-Präparate

Auch hier kommt es gelegentlich zu Völlegefühl, Übelkeit und Erbrechen bei Einnahme der Medikamente. Bei Therapiebeginn treten auch häufig Flush-Reaktionen auf, die im Laufe der Therapie dann zurückgehen. Ferner ist eine Verschlechterung der Glukosetoleranz mit einer möglichen Manifestation eines Diabetes mellitus zu erwarten. Ein Anstieg der Harnsäurekonzentration im Serium zu beobachten, was wiederum zu einer höheren Inzidenz an Gichtanfällen führt. Haarausfall, Hauttrockenheit und Pigmentationsstörungen sind beschrieben, ebenso liegen Fallberichte über zentrale Gesichtsfeldausfälle unter Therapie mit Besserung nach Absetzen des Medikaments sowie ein Fallbericht über eine toxische Sehnervenschädigung vor.

Während die gewünschte Wirkung mit den bekannten Mitteln im allgemeinen in ausreichendem Maß erreicht werden kann, ist die Verträglichkeit verbesserungsbedürftig.

Wie nun gefunden wurde, sind für die Behandlung der Hyperlipidämie überraschenderweise die Verbindungen gemäß der deutschen Offenlegungsschriften P 2557425 (bzw. der französischen Anmeldung Nr. 7638135) und der europäischen Patentanmeldung 0113911 besonders geeignet.

Bei den erfindungsgemäß verwendbaren Stoffen handelt es sich insbesondere um die Verbindungen der Formeln I bis IV.

I. Verbindungen der Formel

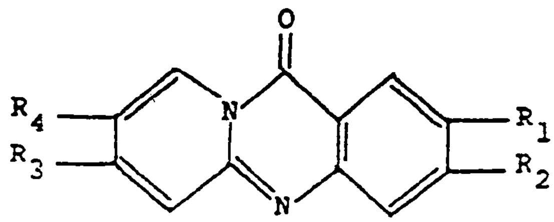 (I)

worin $R_1$ bis $R_4$ folgende Bedeutung haben :

| Verbindung | $R_1$ | $R_2$ | $R_3$ | $R_4$ |
|---|---|---|---|---|
| (a) | COOH | H | H | H |
| (b) | 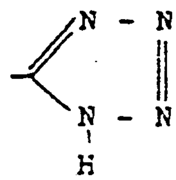 | H | H | H |
| (c) | 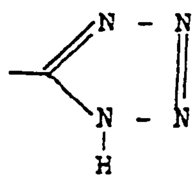 | H | H | $CH(CH_3)_2$ |
| (d) | COOH | H | H | $CH(CH_3)_2$ |
| (e) | 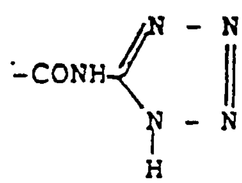 | H | H | $CH(CH_3)_2$ |
| (f) | H | H | H | COOH |
| (g) | $CH_3$ | H | H | COOH |
| (h) | $CH_3O$ | H | H | COOH |

| Verbindung | $R_1$ | $R_2$ | $R_3$ | $R_4$ |
|---|---|---|---|---|
| (i) | Cl | H | H | COOH |
| (j) | Br | H | H | COOH |
| (k) | -CH=CH-CH=CH- | | H | COOH |
| (l) | $C_2H_5O$ | H | H | COOH |
| (m) | $CH(CH_3)_2O$ | H | H | COOH |
| (n) | $CH(CH_3)_2$ | H | H | COOH |
| (o) | COOH | H | H | $CH_3$ |
| (p) | $CH(CH_3)_2$ | H | H | 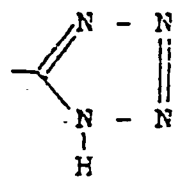 |

Die gesamte Gruppe

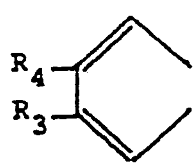

kann auch durch die Gruppe

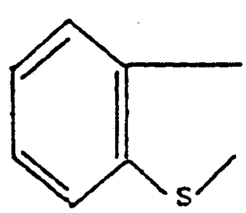

ersetzt sein, während $R_1$ COOH, $R_2$ H ist (q)

Die meisten hier und im folgenden genannten erfindungsgemäß verwendbaren Verbindungen sind bekannt, z.B. aus DE-A 2557425, 2645110, 2812586 und 3315471. Anderenfalls können die Verbindungen, etwa Verbindung (c) und Verbindung (q), analog den dort angegebenen Methoden erhalten werden.

Elementaranalyse :

| | | % C | % H | % N | % S |
|---|---|---|---|---|---|
| (c) | ber.: | 62,74 | 4,57 | 27,45 | - |
| | gef.: | 62,51 | 4,30 | 27,19 | - |
| | (Schmelzpunkt 251°C (Zers.) | | | | |
| (g) | ber.: | 60,81 | 2,70 | 9,46 | 10,82 |
| | gef.: | 60,99 | 2,80 | 9,26 | 10,80 |

II. Verbindungen der Formel

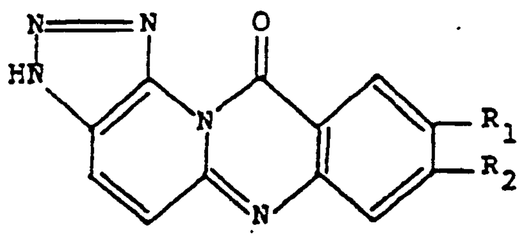

(II)

worin $R_1$ und $R_2$ folgende Bedeutung haben :

| Verbindung | $R_1$ | $R_2$ |
|---|---|---|
| (a) | H | H |
| (b) | $CH(CH_3)_2$ | H |
| (c) | $CH_3$ | H |
| (d) | Cl | H |
| (e) | $CH_3O$ | H |
| (f) | $CH(CH_3)_2O$ | H |
| (g) | -CH=CH-CH=CH- | |

III Verbindungen der Formel

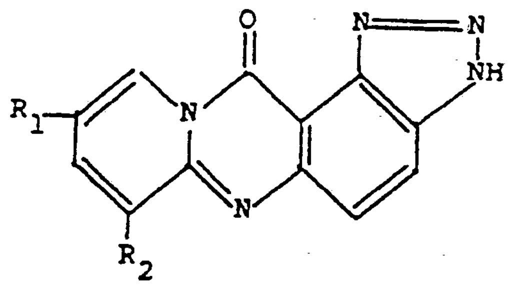

(III)

worin $R_1$ und $R_2$ folgende Bedeutung haben :

| Verbindung | $R_1$ | $R_2$ |
|---|---|---|
| (a) | H | H |
| (b) | $CH_3$ | H |
| (c) | $CH(CH_3)_2$ | H |
| (d) | H | $CH_3$ |

IV. Verbindungen der Formel

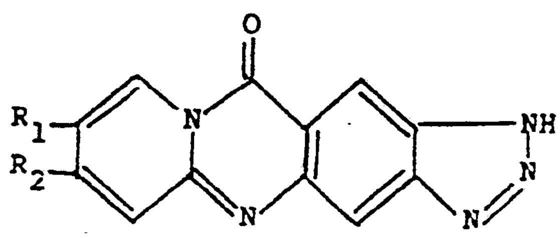

(IV)

worin $R_1$ und $R_2$ folgende Bedeutung haben :

| Verbindung | $R_1$ | $R_2$ |
|---|---|---|
| (a) | H | H |
| (b) | $CH_3$ | H |
| (c) | $CH(CH_3)_2$ | H |

Die gesamte Gruppe

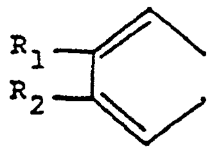

kann auch

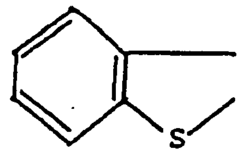

bedeuten (Verbindung (d)).

Die meisten Verbindungen der Gruppen II bis IV sind z.B. aus der europäischen Patentanmeldung 113911 bekannt. Die neuen Verbindungen III(c), III(d), IV(c) und IV(d) können analog erhalten werden.

Elementaranalyse :

| | | % C | % H | % N | % S |
|---|---|---|---|---|---|
| III(c) | ber.: | 64,50 | 4,69 | 25,08 | - |
| | gef.: | 64,11 | 4,44 | 25,23 | - |
| III(d) | ber.: | 62,15 | 3,61 | 27,89 | - |
| | gef.: | 61,16 | 3,42 | 27,35 | - |
| IV(c) | ber.: | 64,50 | 4,69 | 25,08 | - |
| | gef.: | 64,73 | 4,31 | 24,76 | - |
| IV(d) | ber.: | 57,33 | 2,39 | 23,89 | 10,93 |
| | gef.: | 57,74 | 2,16 | 23,50 | 10,62 |

Formulierungsbeispiele

1. Tabletten

| | |
|---|---|
| 11-Oxo-11-H-pyrido[2,1-b]-chinazolin-2-carbonsäure | 100 g |
| kolloidale Kieselsäure | 10 g |
| Milchzucker | 118 g |
| Kartoffelstärke | 60 g |
| Polyvinylpyrrolidon | 6 g |
| Natrium-Celluloseglykolat | 4 g |
| Magnesiumstearat | 2 g |

Die Bestandteile werden in üblicher Weise zu Tabletten von 300 mg verarbeitet.

2. Kapseln

| | |
|---|---|
| Wirkstoff nach Formel I, II, III oder IV | 300 g |
| Maisstärke | 100 g |

Die Bestandteile werden gut vermischt und die Mischung in 400 mg-Portionen in Gelatine-Steckkapseln abgefüllt.

Die obigen Verbindungen der Formeln I bis IV sind bereits als Arzneistoffe beschrieben worden. Als Anwendungsgebiete wurden angegeben : Prophylaxe und Behandlung allergischer Krankheiten wie Asthma, Heufieber, Conjunktivitis, Urticaria, Ekzeme, Dermatitis. Ferner wurde eine muskelrelaxierende (bronchodilatorische) und vasodilatorische Wirkung erwähnt.

Ein Hinweis auf eine lipidsenkende Wirkung findet sich nicht, und eine solche Wirkung wird durch die bekannten Wirkungen auch nicht nahegelegt.

Gegenüber den bekannten Mitteln zur Behandlung der Hyperlipidämie haben die erfindungsgemäß verwendbaren Verbindungen, etwa die 11-Oxo-11-H pyrido-[2,1-b]-chinazolin-2-carbonsäure und ihre Salze, den Vorzug besserer Verträglichkeit. So wurden bei der Behandlung von über 1000 Patienten mit dieser Verbindung in einem anderen Indikationsgebiet keinerlei präparatespezifischen Nebenwirkungen festgestellt.

Die $LD_{50}$ (Maus) ist niedrig. Verbindungen der Formel I zeigen oral eine $LD_{50}$ von mehr als 3000 mg/kg, i.v. mehr als 800 mg/kg. Für die meist stärker lipidsenkenden Verbindungen der Formeln II, III und IV ist die orale $LD_{50}$ an der Maus über 300 mg/kg,

Für die Anwendung werden die Verbindungen in üblicher Weise zu gebräuchlichen galenischen Zubereitungen verarbeitet. Deutlich im Vordergrund steht die orale Anwendung, vorzugsweise in Form von Kapseln ; aber auch alle anderen oralen Darreichungsformen kommen in Betracht, also beispielsweise Tabletten, Dragées, Granulate, Suspensionen, Retardformen.

Die Dosis pro Tag beträgt etwa 100-1000 mg ; sie kann in 1-3 Einzeldosen verabreicht werden.

Mit 600 mg pro Tag ließ sich z.B. bei Verwendung der 11-Oxo-11-H-pyrido[2,1-b]chinazolin-2-carbonsäure beim Menschen der Gesamtcholesterinspiegel um etwa 25% senken.

In einer anderen Studie erhielten 8 gesunde Probanden eine definierte purinreiche Diät über die Prüfdauer von 8 Tagen. Nach 4 Tagen wurde mit der Verabreichung von 11-Oxo-11-H-pyrido[2,1-b]-chinazolin-2-carbonsäure (600 mg/Tag) begonnen.

Gesamtcholesterin ($\bar{x}$) ; n = 8

| | |
|---|---|
| Bei Beginn (vor Diät): | 201 ± 48 mg/dl |
| nach 4 Tagen (vor Therapie) | 196 ± 70 mg/dl |
| nach 8 Tagen (nach Therapieende): | 160 ± 48 mg/dl |

Das Ergebnis bedeutet eine 18 prozentige Senkung des Gesamtcholesterins.

Im Versuch an Ratten wurde mit 100 mg/kg pro Tag (in zwei gleichen Gaben) eine deutliche Senkung des Triglyceridspiegels erreicht, z.B. 34% Senkung gegenüber der Kontrolle bei normolipämischen Ratten. Vorteilhafterweise wurde gleichzeitig der Harnsäurespiegel um 38% gesenkt. Es läßt sich daher mit einer Substanz eine Therapie der Hyperlipidämie und der Hyperurikämie durchführen. Dadurch können unerwünschte Wechselwirkungen, wie sie bei der sonst nötigen Behandlung mit je einem Wirkstoff gegen die Hyperurikämie und gegen die Hyperlipidämie auftreten können, von vornherein vermieden werden.

## Patentansprüche

1. Verwendung von Verbindungen, gegebenenfalls in Salzform, der folgenden Formeln

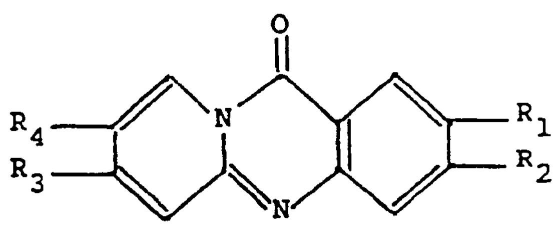

(I)

worin die Substituenten $R_1$, $R_2$, $R_3$ und $R_4$ in dieser Reihenfolge für

- (a) COOH/H/H/H,
- (b) Tetrazol-5-yl/H/H/H,
- (c) Tetrazol-5-yl/H/H/i-Propyl,
- (d) COOH/H/H/i-Propyl,
- (e) Tetrazol-5-yl-NHCO/H/H/i-Propyl,
- (f) H/H/H/COOH,
- (g) $CH_3$/H/H/COOH,
- (h) $CH_3O$/H/H/COOH,
- (i) Cl/H/H/COOH,
- (j) Br/H/H/COOH,
- (k) $R_1 + R_2$ gleich —CH=CH-CH=CH-/H/COOH,
- (l) $C_2H_5O$/H/H/COOH,
- (m) $OCH(CH_3)_2$/H/H/COOH,
- (n) $CH(CH_3)_2$/H/H/COOH,
- (o) COOH/H/H/$CH_3$,
- (p) $CH(CH_3)_2$/H/H/Tetrazol-5-yl oder
- (q) die gesamte Gruppe

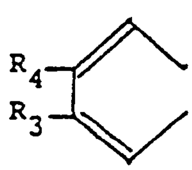

für

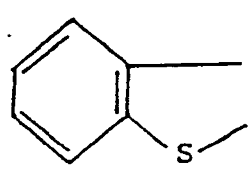

steht, oder

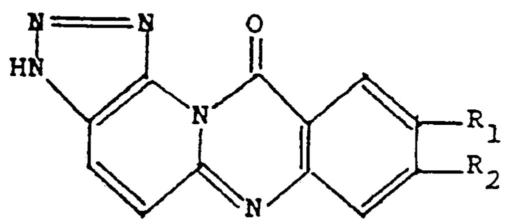

(II)

worin die Substituenten $R_1/R_2$ für

(a) H/H,
(b) $CH(CH_3)_2/H$,
(c) $CH_3/H$,
(d) Cl/H,
(e) $CH_3O/H$.
(f) $CH(CH_3)_2O/H$ oder
(g) gemeinsam für —CH=CH-CH=CH— stehen, oder

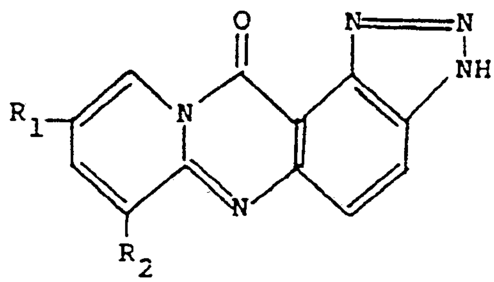

(III)

wobei die Substituenten $R_1/R_2$ für

(a) H/H,
(b) $CH_3/H$,
(c) $CH(CH_3)_2/H$ oder
(d) $H/CH_3$
stehen, oder

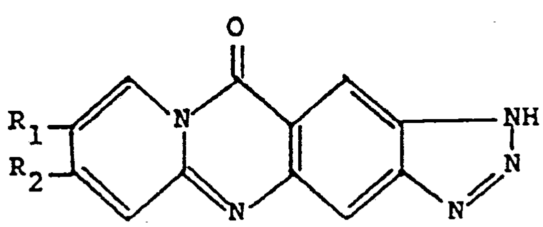

(IV)

worin die Substituenten $R_1/R_2$ für

(a) H/H
(b) $CH_3/H$ oder
(c) $CH(CH_3)_2/H$ stehen,

zur Herstellung von Arzneimitteln für die Behandlung der Hyperlipidämie.

2. Verwendung gemäß Anspruch 1 dadurch gekennzeichnet daß die Verbindung von 11-Oxo-11-H-pyrido[2,1-b]-chinazolin-2-carbonsäure oder eines ihrer Salzen ist zur Herstellung von Arzneimitteln für die Behandlung der Hyperlipidämie.

## Claims

1. Use of compounds, optionally in salt form, of the following formulae

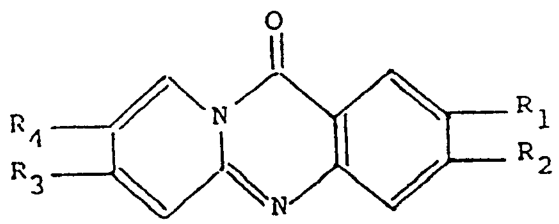

(I)

wherein the substituents $R_1$, $R_2$, $R_3$ and $R_4$ in this order represent

(a) COOH/H/H/H,
(b) Tetrazol-5-yl/H/H/H,
(c) Tetrazol-5-yl/H/H/i-propyl,
(d) COOH/H/H/i-propyl,
(e) Tetrazol-5-yl-NHCO/H/H/i-propyl,
(f) H/H/H/COOH,
(g) $CH_3$/H/H/COOH,
(h) $CH_3O$/H/H/COOH,
(i) Cl/H/H/COOH,
(j) Br/H/H/COOH,
(k) $R_1$ + $R_2$ equals —CH=CH-CH=CH-/H/COOH,
(l) $C_2H_5O$/H/H/COOH,
(m) $OCH(CH_3)_2$/H/H/COOH,
(n) $CH(CH_3)_2$/H/H/COOH,
(o) COOH/H/H/$CH_3$,
(p) $CH(CH_3)_2$/H/H/Tetrazol-5-yl or
(q) the entire group

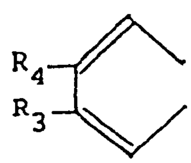 represents 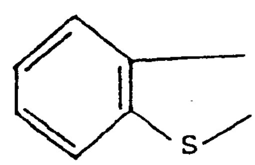

or

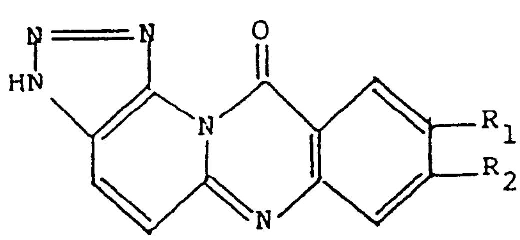

(II)

wherein the substituents $R_1$/$R_2$ represent

(a) H/H,
(b) $CH(CH_3)_2$/H,
(c) $CH_3$/H,
(d) Cl/H,
(e) $CH_3O$/H
(f) $CH(CH_3)_2O$/H or

(g) together represent —CH=CH-CH=CH— or

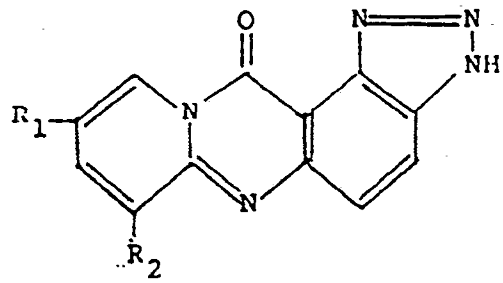

(III)

wherein the substituents $R_1/R_2$ represent

(a) H/H,
(b) $CH_3$/H,
(c) CH $(CH_3)_2$/H or
(d) H/$CH_3$

or

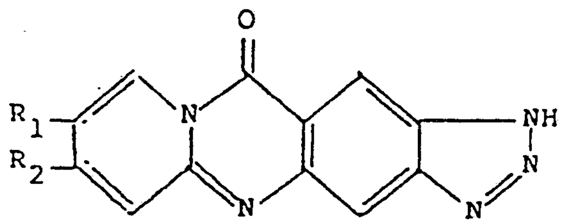

(IV)

wherein the substituents $R_1/R_2$ represent

(a) H/H
(b) $CH_3$/H or
(c) $CH(CH_3)_2$/H,

for the preparation of pharmaceutical compositions for the treatment of hyperlipidaemia.

2. Use according to claim 1, characterised in that the compound is 11-oxo-11-H-pyrido[2,1-b]quinazolin-2-carboxylic acid or one of the salts thereof, for the preparation of pharmaceutical compositions for the treatment of hyperlipidaemia.

## Revendications

1. Utilisation de composés, éventuellement sous forme de leurs sels, des formules suivantes

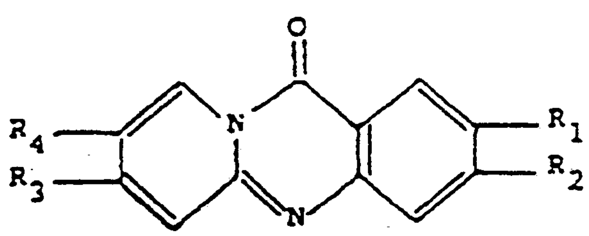

(I)

où les substituants $R_1$, $R_2$, $R_3$ et $R_4$ ont pour signification dans cette série

(a) COOH/H/H/H,
(b) Tetrazol-5-yl/H/H/H,
(c) Tetrazol-5-yl/H/H/i-propyle,
(d) COOH/H/H/i-propyle,

(e) Tetrazol-5-yl-NHCO/H/H/i-propyle,
(f) H/H/H/COOH,
(g) $CH_3$/H/H/COOH,
(h) $CH_3O$/H/H/COOH,
(i) Cl/H/H/COOH,
(j) Br/H/H/COOH,
(k) $R_1 + R_2$ identiques à —CH=CH-CH=CH-/H/COOH,
(l) $C_2H_5O$/H/H/COOH,
(m) $OCH(CH_3)_2$/H/H/COOH,
(n) $CH(CH_3)_2$/H/H/COOH,
(o) COOH/H/H/$CH_3$,
(p) $CH(CH_3)_2$/H/H/tetrazol-5-yl ou
(q) le groupe total

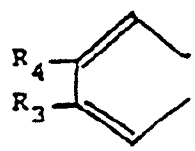

pour

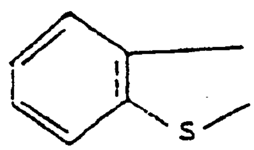

ou

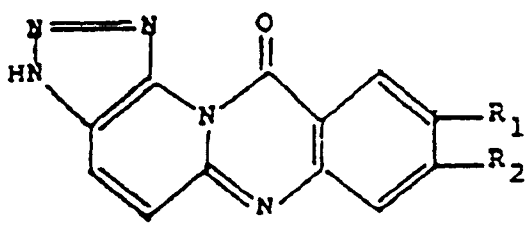

(II)

où les substituants $R_1$/$R_2$ ont pour signification

(a) H/H,
(b) $CH(CH_3)_2$/H,
(c) $CH_3$/H,
(d) Cl/H,
(e) $CH_3O$/H.
(f) $CH(CH_3)_2O$/H ou
(g) conjointement pour —CH=CH-CH=CH—, ou

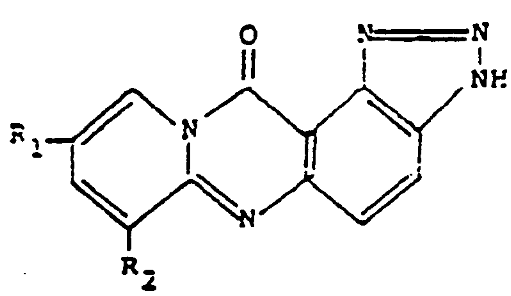

(III)

où les substituants $R_1/R_2$ ont pour signification

(a) H/H,
(b) $CH_3/H$,
(c) $CH(CH_3)_2/H$ ou
(d) $H/CH_3$
ou

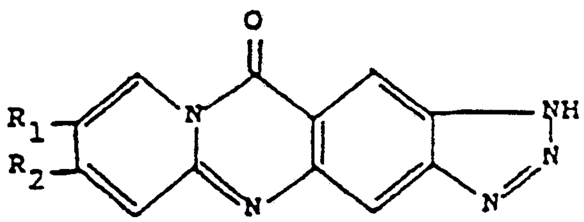

(IV)

où les substituants $R_1/R_2$ ont pour signification

(a) H/H,
(b) $CH_3/H$ ou
(c) $CH(CH_3)_2/H$,

pour la préparation de médicaments destinés au traitement de l'hyperlipidémie.

2. Utilisation selon la revendication 1, caractérisée en ce que le composé de l'acide 11-oxo-11-H-pyrido[2,1-b]-quinazolin-2-carboxylique ou l'un de ses sels destiné à la préparation de médicaments pour le traitement de l'hyperlipidémie.